# EUROPEAN PATENT SPECIFICATION

(11) **EP 3 469 929 B1**
(45) Date of publication and mention of the grant of the patent: **25.03.2026**
(21) Application number: 16913424.4
(22) Date of filing: 16.12.2016
(51) Int. Cl.: A24F 40/05, A24F 40/44, A61M 15/00, A61M 11/00, A61M 15/06

(54) **ATOMIZER AND ELECTRONIC CIGARETTE COMPRISING SAME**
ZERSTÄUBER UND ELEKTRONISCHE ZIGARETTE DAMIT
ATOMISEUR ET CIGARETTE ÉLECTRONIQUE COMPRENANT CELUI-CI

(30) Priority: 18.08.2016 CN 201620898460 U
(43) Date of publication of application: 17.04.2019
(73) Proprietor: China Tobacco Hunan Industrial Co., Ltd., Changsha, Hunan 410007 (CN)
(72) Inventor: LIU, Jianfu, Changsha Hunan 410007 (CN); ZHONG, Kejun, Changsha Hunan 410007 (CN); GUO, Xiaoyi, Changsha Hunan 410007 (CN); HUANG, Wei, Changsha Hunan 410007 (CN); YU, Hong, Changsha Hunan 410007 (CN); DAI, Yuangang, Changsha Hunan 410007 (CN); YIN, Xinqiang, Changsha Hunan 410007 (CN); YI, Jianhua, Changsha Hunan 410007 (CN); ZHOU, Yongquan, Changsha Hunan 410007 (CN)
(74) Representative: Alpspitz IP
(86) International application number: PCT/CN2016/110394
(87) International publication number: WO 2018/032672

(56) References cited:
- EP-A1- 3 278 678
- CN-A- 104 382 238
- CN-A- 105 795 526
- CN-A- 105 795 526
- CN-A- 105 795 527
- CN-A- 105 795 527
- CN-U- 203 040 683
- CN-U- 203 662 023
- CN-U- 203 662 023
- CN-U- 205 432 145
- CN-U- 205 432 145
- US-A1- 2014 251 325
- US-A1- 2018 352 854

## Description

### Field of Invention

The present invention belongs to an atomizer and an electronic cigarette.

### Background of Invention

CN 203 662 023 U discloses an ultrasonic atomizing device. CN 205 432 145 U discloses an ultrasonic atomizer and electronic cigarette. CN 104 382 238 A discloses an electromagnetic induction smoke generating device and an electronic cigarette with the device. CN 105 795 527 A discloses an electronic cigarette atomizer and an electronic cigarette. CN 105 795 526 A discloses an electronic cigarette atomizer.

In an existing ultrasonic high-frequency atomizing electronic cigarette, the surface of an ultrasonic high-frequency atomizing piece is covered with a piece of tobacco tar guide cotton, and the contact between the tobacco tar guide cotton and the atomizing piece is surface contact.

For the atomizing piece made by piezoelectric ceramic, after the piezoelectric ceramic piece is energized, it does not immediately oscillate to atomize the tobacco tar guide cotton. Instead, it is energized at first to generate heat, and when the heat reaches a certain level, the piezoelectric ceramic piece oscillates to atomize the tobacco tar guide cotton. After the tobacco tar guide cotton is covered on the surface of the piezoelectric ceramic piece and the piezoelectric ceramic piece is energized, the piezoelectric ceramic piece generates heat, but since the tobacco tar guide cotton itself absorbs the heat and the contact surface between the tobacco tar guide cotton and the piezoelectric ceramic piece is large, a part of the heat is transferred to the tobacco tar guide cotton, such that the piezoelectric ceramic piece takes a long time to produce the heat needed to achieve its oscillation. In this way, the duration from the time when piezoelectric ceramic piece starts working to the start of oscillation atomization is too long, and the waiting time of a user to inhale the first mouth of smoke is long, so that the experience is poor.

Moreover, since the contact between the tobacco tar guide cotton and the atomizing piece is surface contact the phenomenon that the tobacco tar guide cotton is difficult to atomize after being supersaturated due to too much tobacco tar absorption is often generated.

### Summary of Invention

In view of the above problems, the present disclosure aims at providing an atomizer with a high smoke production speed and a good atomizing effect, and an electronic cigarette.

According to the present invention, an atomizer with the features of claim 1 is provided.

According to the present teaching, there is provided an atomizer, including an atomizer body, and an atomizing core and a tobacco tar cavity both arranged in the atomizer body, wherein the atomizing core includes an atomizing piece and a strip-shaped tobacco tar guide body which guides tobacco tar in the tobacco tar cavity to an atomizing surface of the atomizing piece; and
the strip-shaped tobacco tar guide body spreads across the atomizing piece and props against the atomizing surface of the atomizing piece, and the area of a contact surface of the strip-shaped tobacco tar guide body and the atomizing piece is less than the area of the atomizing surface of the atomizing piece.

Accordingly, the area of the contact surface of the strip-shaped tobacco tar guide body and the atomizing piece is less than the area of the atomizing surface of the atomizing piece, which is similar to the situation of line contact, so that the strip-shaped tobacco tar guide body scarcely absorbs the heat generated by the atomizing piece, the atomizing piece can quickly generate heat and start oscillating, and thus the first mouth of smoke is quickly atomized. In the solution, the time from the start of working of the atomizing piece to the start of oscillation atomization is shortened, such that smoke is released quicker.

Specifically, the atomizer body includes a shell, an air inlet, a suction nozzle installed at the top of the shell, and a connecting electrode installed at the bottom of the shell, and the tobacco tar cavity and the atomizing core are arranged in the shell;
an air inlet passage communicated with the air inlet is arranged in the shell, and the air inlet passage leads to the atomizing surface of the atomizing piece; and an air outlet passage communicated with the suction nozzle is arranged in the shell;
and the atomizing piece is electrically connected with the connecting electrode.

Further, the atomizing core further includes a fixing sleeve arranged to lean against the inner wall of the shell and located below the tobacco tar cavity, and a first hollow cavity is arranged in the fixing sleeve; and
an atomizing piece fixing seat is arranged at the lower part of the first hollow cavity, and the atomizing piece is installed on the atomizing piece fixing seat; and the atomizing surface of the atomizing piece props against the strip-shaped tobacco tar guide body, and a non-atomizing surface of the atomizing piece is electrically connected with the connecting electrode.

The air inlet is located at the bottom of the connecting electrode, the air inlet passage is a second hollow cavity arranged in the connecting electrode, and the second hollow cavity is communicated with the first hollow cavity; and
the air outlet passage includes an air pipe, the top of the air pipe is communicated with the suction nozzle, a hollow sealing cover is arranged at the lower end of the air pipe, and the sealing cover is connected with the top of the first hollow cavity and seals the top of the first hollow cavity.

After the sealing cover is connected with the first hollow cavity of the fixing sleeve, an isolation surface is formed by the seal, and the tobacco tar cavity is located above an upper surface of the sealing cover.

According to the present invention, fixing grooves are symmetrically formed in the outer wall of the fixing sleeve, via holes are laterally formed in the lower ends of the fixing grooves in the outer wall of the fixing sleeve, the strip-shaped tobacco tar guide body penetrates through the via holes, the middle segment of the strip-shaped tobacco tar guide body props against the atomizing surface of the atomizing piece, and the remaining segments of both ends of the strip-shaped tobacco tar guide body are clamped in the fixing grooves; and
the both ends of the strip-shaped tobacco tar guide body stretch from the fixing grooves and are inserted into the tobacco tar cavity.

Accordingly, the tobacco tar in the tobacco tar cavity is directly guided by the strip-shaped tobacco tar guide body into the atomizing piece.

In another specific embodiment, not forming part of the claimed invention, a tobacco tar inlet groove is formed in the edge of the sealing cover, annular tobacco tar guide cotton is arranged to lean against the inner wall of the fixing sleeve, and the tobacco tar guide cotton props against the tobacco tar inlet groove; and
a plurality of strip-shaped tobacco tar guide bodies arranged at intervals spread across the atomizing piece, and both ends of each strip-shaped tobacco tar guide body prop against the tobacco tar guide cotton.

Accordingly, the strip-shaped tobacco tar guide body is not directly in contact with the tobacco tar cavity, but the tobacco tar is indirectly transferred to the strip-shaped tobacco tar guide body through the tobacco tar guide cotton and then enters the atomizing piece, and the process effectively slows the tobacco tar leakage phenomenon resulting from that the tobacco tar is directly absorbed by the strip-shaped tobacco tar guide body.

In order to facilitate the fixing of the strip-shaped tobacco tar guide body, a fixing bayonet is formed in the atomizing piece fixing seat, and the corner of the strip-shaped tobacco tar guide body is clamped and fixed in the fixing bayonet.

Preferably, the strip-shaped tobacco tar guide body is a glass fiber thread having a diameter of 0.3 mm to 5 mm. Since the diameter of the glass fiber thread is relatively small, the glass fiber thread absorbs less tobacco tar when the atomizing piece is not working, therefore the phenomenon can be avoided that little smoke is generated or the smoke is produced slowly because the surface of the atomizing piece is immersed by too much tobacco tar.

Preferably, the area of the contact surface of the strip-shaped tobacco tar guide body and the atomizing piece accounts for 5% to 25% of the area of the atomizing surface of the atomizing piece.

Preferably, the atomizing piece is a solid piezoelectric ceramic piece.

Correspondingly, the present teachings further provide an electronic cigarette, including an external power source, wherein the electronic cigarette further includes the atomizer in the above solution, and the external power source is connected with the connecting electrode of the atomizer.

The present teachings have the following significant effects:
1. The area of the contact surface of the strip-shaped tobacco tar guide body and the atomizing piece is less than the area of the atomizing surface of the atomizing piece, which is similar to the structural form of line contact, so that the strip-shaped tobacco tar guide body scarcely absorbs the heat generated by the atomizing piece, and the atomizing piece can quickly generate heat and start oscillating, and thus the first mouth of smoke is produced by quick atomization. In the solution, the time from the start of working of the atomizing piece to the start of oscillation atomization is shortened, such that smoke is released quicker, and the user experience is better.
2. Due to the small contact area, the tobacco tar transferred by the strip-shaped tobacco tar guide body is not excessive, so that the phenomenon that the tobacco tar guide body is difficult to atomize after being supersaturated due to too much tobacco tar absorption is not generated.

### Brief Description of the Drawings

The present teachings are further illustrated below in combination with the drawings.
Fig.1 is a section view of an embodiment 1.
Fig.2 is an A-A section view of Fig.1.
Fig.3 is an explosive view of the embodiment 1.
Fig.4 is a section view of an embodiment 2.
Fig.5 is a B-B section view of Fig.2.
Fig.6 is an explosive view of the embodiment 2.

Reference signs: 1-shell, 2-air inlet, 3-suction nozzle, 4-tobacco tar cavity, 5-air pipe, 6-connecting electrode, 7-fixing sleeve, 8-tobacco tar guide cotton, 51-atomizing piece, 52- strip-shaped tobacco tar guide body, 53-sealing cover, 54-tobacco tar inlet groove, 55-bayonet, 58-atomizing piece fixing seat, 61-second hollow cavity, 71-first hollow cavity, 72 fixing groove, and 73-via hole.

### Detailed Description of the Embodiments

### Embodiment 1

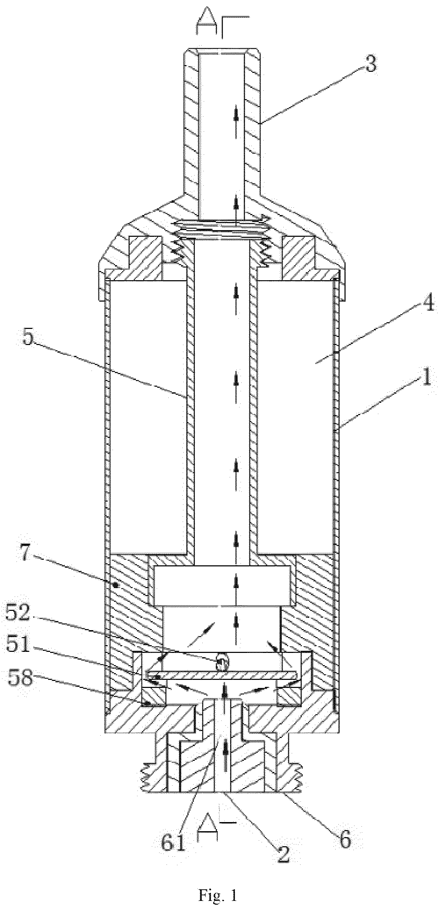

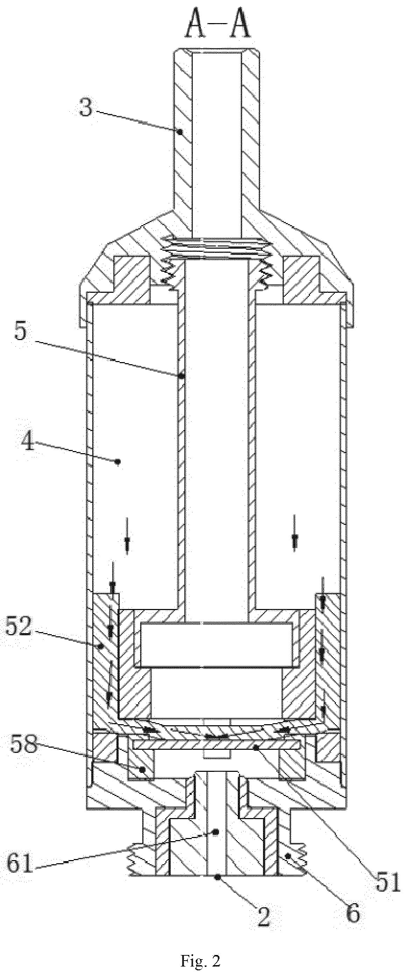

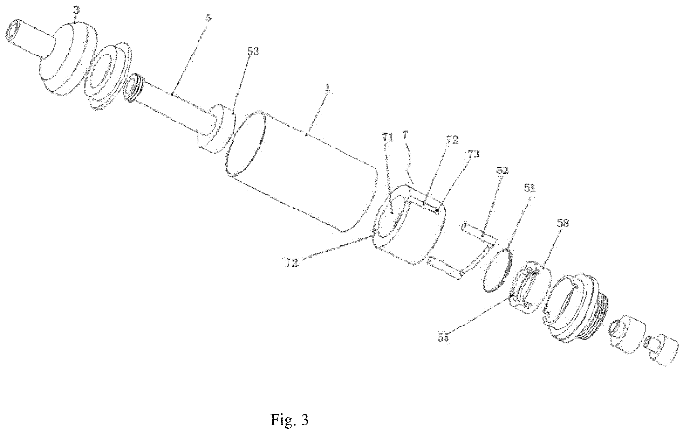

As shown in Fig.1 to Fig.3, an atomizer, including a shell 1, an air inlet 2, a suction nozzle 3 installed at the top of the shell 1, a tobacco tar cavity 4 and an atomizing core both arranged in the shell 1, and a connecting electrode 6 installed at the bottom of the shell 1.

The atomizing core includes an atomizing piece 51 and a strip-shaped tobacco tar guide body 52 which guides tobacco tar in the tobacco tar cavity 4 to an atomizing surface of the atomizing piece 51.

The strip-shaped tobacco tar guide body 52 spreads across the atomizing piece 51 and props against the atomizing surface of the atomizing piece 51, and the area of a contact surface of the strip-shaped tobacco tar guide body 52 and the atomizing piece 51 is less than the area of the atomizing surface of the atomizing piece 51.

An air inlet passage communicated with the air inlet 2 is arranged in the shell 1, and the air inlet passage leads to the atomizing surface of the atomizing piece 51. An air outlet passage communicated with the suction nozzle 3 is arranged in the shell 1.

The atomizing core further includes a fixing sleeve 7 arranged to lean against the inner wall of the shell 1, and the fixing sleeve 7 is located below the tobacco tar cavity 4, and a first hollow cavity 71 is arranged in the fixing sleeve 7. An atomizing piece fixing seat 58 is arranged at the lower part of the first hollow cavity 71. The atomizing piece 51 is installed on the atomizing piece fixing seat 58. The atomizing surface of the atomizing piece 51 props against the strip-shaped tobacco tar guide body 52, and a non-atomizing surface of the atomizing piece 51 is electrically connected with the connecting electrode 6.

The air inlet 2 is located at the bottom of the connecting electrode 6. The air inlet passage is a second hollow cavity 61 arranged in the connecting electrode 6, and the second hollow cavity 61 is communicated with the first hollow cavity 71.

The air outlet passage includes an air pipe 5, the top of the air pipe 5 is communicated with the suction nozzle 3, and a hollow sealing cover 53 is arranged at the lower end of the air pipe 5. The sealing cover 53 is connected with the top of the first hollow cavity 71 and seals the top of the first hollow cavity.

Fixing grooves 72 are symmetrically formed in the outer wall of the fixing sleeve 7. Via holes 73 are laterally formed in the lower ends of the fixing grooves 72 in the outer wall of the fixing sleeve 7, the strip-shaped tobacco tar guide body 52 penetrates through the via holes 73, the middle segment of the strip-shaped tobacco tar guide body 52 props against the atomizing surface of the atomizing piece 51, and the remaining segments of both ends of the strip-shaped tobacco tar guide body 52 are clamped in the fixing grooves 72. The both ends of the strip-shaped tobacco tar guide body 52 stretch from the fixing grooves 72 and are inserted into the tobacco tar cavity 4. A fixing bayonet 55 is formed in the atomizing piece fixing seat 58, and the corner of the strip-shaped tobacco tar guide body 52 is clamped and fixed in the fixing bayonet 55.

The strip-shaped tobacco tar guide body 52 is preferably a glass fiber thread having a diameter of 0.3 mm to 5 mm.

The atomizing piece 51 is preferably a solid piezoelectric ceramic piece.

Preferably, the area of the contact surface of the strip-shaped tobacco tar guide body 52 and the atomizing piece 51 accounts for 5% to 25% of the area of the atomizing surface of the atomizing piece 51.

Correspondingly, an electronic cigarette is further provided, including an external power source, wherein the electronic cigarette further includes the atomizer in the above solution, and the external power source is connected with the connecting electrode 6 of the atomizer.

### Embodiment 2 (not forming part of the claimed invention)

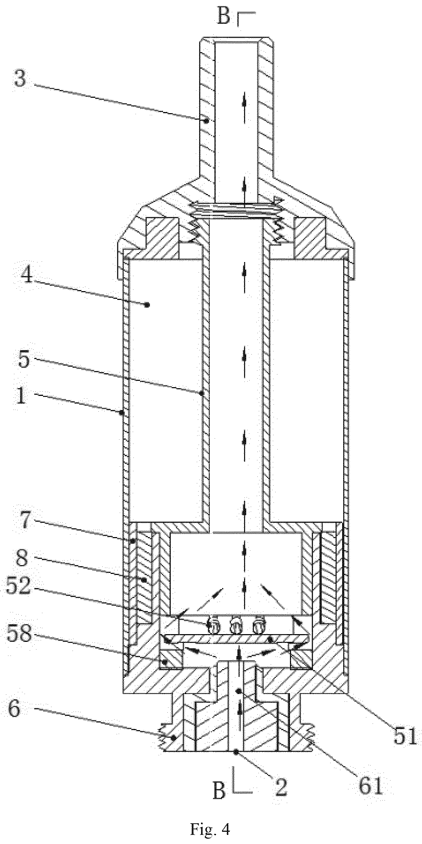

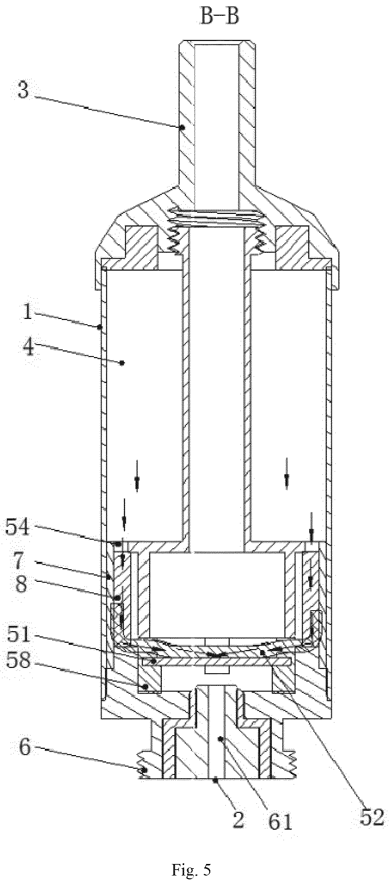

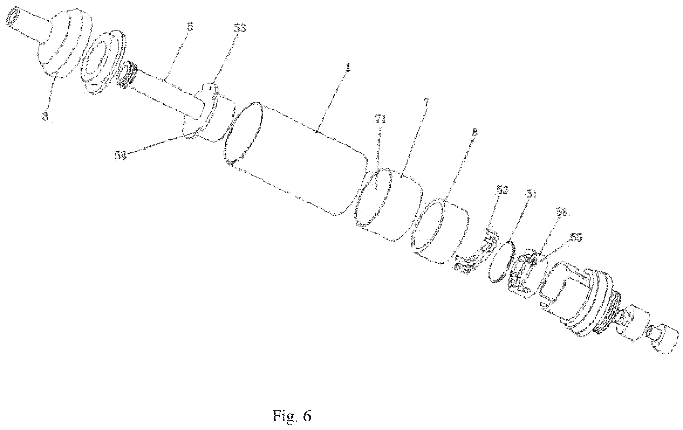

As shown in Fig.4 to Fig.6, an atomizer, including a shell 1, an air inlet 2, a suction nozzle 3 installed at the top of the shell 1, a tobacco tar cavity and an atomizing core which are both arranged in the shell 1, and a connecting electrode 6 installed at the bottom of the shell 1.

The atomizing core includes an atomizing piece 51 and a strip-shaped tobacco tar guide body 52 which guides tobacco tar in the tobacco tar cavity 4 to an atomizing surface of the atomizing piece 51.

The strip-shaped tobacco tar guide body 52 spreads across the atomizing piece 51 and props against the atomizing surface of the atomizing piece 51, and the area of a contact surface of the strip-shaped tobacco tar guide body 52 and the atomizing piece 51 is less than the area of the atomizing surface of the atomizing piece 51.

An air inlet passage communicated with the air inlet 2 is arranged in the shell 1, and the air inlet passage leads to the atomizing surface of the atomizing piece 51. An air outlet passage communicated with the suction nozzle 3 is arranged in the shell 1.

The atomizing core further includes a fixing sleeve 7 arranged to lean against the inner wall of the shell 1, the fixing sleeve 7 is located below the tobacco tar cavity 4, and a first hollow cavity 71 is arranged in the fixing sleeve 7. An atomizing piece fixing seat 58 is arranged at the lower part of the first hollow cavity 71. The atomizing piece 51 is installed on the atomizing piece fixing seat 58. The atomizing surface of the atomizing piece 51 props against the strip-shaped tobacco tar guide body 52, and a non-atomizing surface of the atomizing piece 51 is electrically connected with the connecting electrode 6.

The air inlet 2 is located at the bottom of the connecting electrode 6. The air inlet passage is a second hollow cavity 61 arranged in the connecting electrode 6, and the second hollow cavity 61 is communicated with the first hollow cavity 71.

The air outlet passage includes an air pipe 5, the top of the air pipe 5 is communicated with the suction nozzle 3, and a hollow sealing cover 53 is arranged at the lower end of the air pipe 5. The sealing cover 53 is connected with the top of the first hollow cavity 71 and seals the top of the first hollow cavity 71.

A tobacco tar inlet groove 54 is formed in the edge of the sealing cover 53, annular tobacco tar guide cotton 8 is arranged to lean against the inner wall of the fixing sleeve 7, and the tobacco tar guide cotton 8 props against the tobacco tar inlet groove 54.

A plurality of strip-shaped tobacco tar guide bodies 52 arranged at intervals spread across the atomizing piece 51, and both ends of each strip-shaped tobacco tar guide body 52 are clamped between the tobacco tar guide cotton 8 and the fixing sleeve 7, so that the both ends of each strip-shaped tobacco tar guide body 52 prop against the tobacco tar guide cotton 8. A fixing bayonet 55 is formed in the atomizing piece fixing seat 58, and the corner of the strip-shaped tobacco tar guide body 52 is clamped and fixed in the fixing bayonet 55.

The strip-shaped tobacco tar guide body 52 is preferably a glass fiber thread having a diameter of 0.3 mm to 5 mm.

The atomizing piece 51 is preferably a solid piezoelectric ceramic piece.

Preferably, the area of the contact surface of the strip-shaped tobacco tar guide body 52 and the atomizing piece 51 accounts for 5% to 25% of the area of the atomizing surface of the atomizing piece.

Correspondingly, an electronic cigarette is further provided, including an external power source, wherein the electronic cigarette further includes the atomizer in the above solution, and the external power source is connected with the connecting electrode 6 of the atomizer.

## Claims

1. An atomizer, comprising an atomizer body, and an atomizing core and a tobacco tar cavity (4) both arranged in the atomizer body, wherein the atomizing core comprises an atomizing piece (51) and a strip-shaped tobacco tar guide body (52) which guides tobacco tar in the tobacco tar cavity (4) to an atomizing surface of the atomizing piece (51); and
the strip-shaped tobacco tar guide body (52) spreads across the atomizing piece (51) and props against the atomizing surface of the atomizing piece (51), and the area of a contact surface of the strip-shaped tobacco tar guide body (52) and the atomizing piece (51) is less than the area of the atomizing surface of the atomizing piece (51),
wherein
the atomizer body comprises a shell (1), an air inlet (2), a suction nozzle (3) installed at the top of the shell (1), and a connecting electrode (6) installed at the bottom of the shell (1), and the tobacco tar cavity (4) and the atomizing core are arranged in the shell (1);
an air inlet passage communicated with the air inlet (2) is arranged in the shell (1), and the air inlet passage leads to the atomizing surface of the atomizing piece (51); and an air outlet passage communicated with the suction nozzle (3) is arranged in the shell (1); and
the atomizing piece (51) is electrically connected with the connecting electrode (6),
**characterized in that**
the atomizing core further comprises a fixing sleeve (7) arranged to lean against the inner wall of the shell (1) and located below the tobacco tar cavity (4), and a first hollow cavity (71) is arranged in the fixing sleeve (7); and
an atomizing piece fixing seat (58) is arranged at the lower part of the first hollow cavity (71), and the atomizing piece (51) is installed on the atomizing piece fixing seat (58); and the atomizing surface of the atomizing piece (51) props against the strip-shaped tobacco tar guide body (52), and a non-atomizing surface of the atomizing piece (51) is electrically connected with the connecting electrode (6), fixing grooves (72) are symmetrically formed in the outer wall of the fixing sleeve (7), via holes (73) are laterally formed in the lower ends of the fixing grooves (72) in the outer wall of the fixing sleeve (7), the strip-shaped tobacco tar guide body (52) penetrates through the via holes (53), the middle segment of the strip-shaped tobacco tar guide body (52) props against the atomizing surface of the atomizing piece (51), and the remaining segments of both ends of the strip-shaped tobacco tar guide body (52) are clamped in the fixing grooves (72); and
the both ends of the strip-shaped tobacco tar guide body (52) stretch from the fixing grooves (72) and are inserted into the tobacco tar cavity (4).

2. The atomizer according to claim 1, wherein the air inlet (2) is located at the bottom of the connecting electrode (6), the air inlet passage is a second hollow cavity (61) arranged in the connecting electrode (6), and the second hollow cavity (61) is communicated with the first hollow cavity (71); and
the air outlet passage comprises an air pipe (5), the top of the air pipe (5) is communicated with the suction nozzle (3), a hollow sealing cover (53) is arranged at the lower end of the air pipe (5), and the sealing cover (53) is connected with the top of the first hollow cavity (71) and seals the top of the first hollow cavity (71).

3. The atomizer according to claim 1, wherein a fixing bayonet (55) is formed in the atomizing piece fixing seat (58), and the corner of the strip-shaped tobacco tar guide body (52) is clamped and fixed in the fixing bayonet (55).

4. The atomizer according to claim 1, wherein the strip-shaped tobacco tar guide body (52) is a glass fiber thread having a diameter of 0.3 to 5 mm.

5. The atomizer according to any one of claims 1-4, wherein the area of the contact surface of the strip-shaped tobacco tar guide body (52) and the atomizing piece (51) accounts for 5% to 25% of the area of the atomizing surface of the atomizing piece (51).

6. The atomizer according to claim 1, wherein the atomizing piece (51) is a solid piezoelectric ceramic piece.

7. An electronic cigarette, comprising an external power source, wherein the electronic cigarette further comprises the atomizer according to any one of claims 1-6, and the external power source is connected with the connecting electrode (6) of the atomizer.

## Patentansprüche

1. Zerstäuber, enthaltend ein Zerstäubergehäuse, und einen Zerstäubungskern und einen Tabakteerhohlraum (4), die beide in dem Zerstäubergehäuse angeordnet sind, wobei der Zerstäubungskern ein Zerstäubungsstück (51) und einen streifenförmigen Tabakteerführungskörper (52) enthält, der den Tabakteer in dem Tabakteerhohlraum (4) zu einer Zerstäubungsoberfläche des Zerstäubungsstücks (51) leitet; und
der streifenförmige Tabakteerführungskörper (52) sich über das Zerstäubungsstück (51) erstreckt und gegen die Zerstäubungsoberfläche des Zerstäubungsstücks (51) stößt, und das Gebiet einer Berührungsfläche des streifenförmigen Tabakteerführungskörpers (52) und des Zerstäubungsstücks (51) kleiner ist als das Gebiet der Zerstäubungsoberfläche des Zerstäubungsstücks (51), wobei
das Zerstäubergehäuse einen Mantel (1), einen Lufteinlass (2), eine in den oberen Teil des Mantels (1) eingebaute Saugdüse (3) und eine in den unteren Teil des Mantels (1) eingebaute Verbindungselektrode (6) enthält, und der Tabakteerhohlraum (4) und der Zerstäubungskern in dem Mantel (1) angeordnet sind;
eine mit dem Lufteinlass (2) verbundene Lufteinlasspassage in dem Mantel (1) angeordnet ist, und die Lufteinlasspassage zur Zerstäubungsoberfläche des Zerstäubungsstücks (51) führt; und eine mit der Saugdüse (3) verbundene Luftauslasspassage in dem Mantel (1) angeordnet ist; und
das Zerstäubungsstück (51) elektrisch mit der Verbindungselektrode (6) verbunden ist,
**dadurch gekennzeichnet dass**
der Zerstäubungskern weiter eine Fixiermanschette (7) enthält, die angeordnet ist zum sich Lehnen gegen die Innenwand des Mantels (1) und die sich unter dem Tabakteerhohlraum (4) befindet , und ein erster hohler Hohlraum (71) in der Fixiermanschette (7) angeordnet ist; und
ein Zerstäubungsstück-Fixiersitz (58) an dem unteren Teil des ersten hohlen Hohlraums (71) angeordnet ist, und das Zerstäubungsstück (51) auf dem Zerstäubungsstück-Fixiersitz (58) eingebaut ist; und die Zerstäubungsoberfläche des Zerstäubungsstücks (51) gegen den streifenförmigen Tabakteerführungskörper (52) stößt, und eine nicht-zerstäubende Oberfläche des Zerstäubungsstücks (51) elektrisch mit der Verbindungselektrode (6) verbunden ist,
Fixiernuten (72) symmetrisch in der Außenwand der Fixiermanschette (7) ausgebildet sind, Durchgangslöcher (73) in den unteren Enden der Fixiernuten (72) seitlich in der Außenwant der Fixiermanschette (7) ausgebildet sind, der streifenförmige Tabakteerführungskörper (52) durch die Durchgangslöcher (53) hindurchtritt, der Mittenabschnitt des streifenförmigen Tabakteerführungskörpers (52) gegen die Zerstäubungsoberfläche des Zerstäubungsstücks (51) stößt, und die restlichen Abschnitte beider Enden des streifenförmigen Tabakteerführungskörpers (52) in den Fixiernuten (72) festgeklemmt sind; und
die beiden Enden des streifenförmigen Tabakteerführungskörpers (52) sich aus den Fixiernuten (72) erstrecken und in den Tabakteerhohlraum (4) eingeführt sind.

2. Zerstäuber nach Anspruch 1, wobei sich der Lufteinlass (2) an der Unterseite der Verbindungselektrode (6) befindet, die Lufteinlasspassage ein in der Verbindungselektrode angeordneter zweiter hohler Hohlraum (61) ist, und der zweite hohle Hohlraum (61) mit dem ersten hohlen Hohlraum (71) verbunden ist; und
die Luftauslasspassage eine Luftröhre (5) enthält, die Oberseite der Luftröhre (5) mit der Saugdüse (3) verbunden ist, eine hohle Dichtungsabdeckung (53) am unteren Ende der Luftröhre (5) angeordnet ist, und die hohle Dichtungsabdeckung (53) mit der Oberseite des ersten hohlen Hohlraums (71) verbunden ist und die Oberseite des ersten hohlen Hohlraums (71) abdichtet.

3. Zerstäuber nach Anspruch 1, wobei ein Fixierbajonett (55) in dem Zerstäubungsstück-Fixiersitz (58) ausgebildet ist, und die Ecke des streifenförmigen Tabakteerführungskörpers (52) in dem Fixierbajonett (55) festgeklemmt und fixiert ist.

4. Zerstäuber nach Anspruch 1, wobei der streifenförmige Tabakteerführungskörper (52) ein Glasfaserfaden mit einem Durchmesser von 0,3 bis 5 mm ist.

5. Zerstäuber nach einem der Ansprüche 1-4, wobei das Gebiet der Berührungsfläche des streifenförmigen Tabakteerführungskörpers (52) und des Zerstäubungsstücks (51) 5% bis 25% des Gebiets der Zerstäubungsoberfläche des Zerstäubungsstücks (51) ausmacht.

6. Zerstäuber nach Anspruch 1, wobei das Zerstäubungsstück (51) ein festes piezoelektrisches Keramikteil ist.

7. Elektronische Zigarette mit einer externen Stromquelle, wobei die elektronische Zigarette weiter einen Zerstäuber nach einem der Ansprüche 1-6 enthält, und die externe Stromquelle mit der Verbindungselektrode (6) des Zerstäubers verbunden ist.

## Revendications

1. Atomiseur, comprenant un corps d'atomiseur et un cœur d'atomisation et une cavité à goudron de tabac (4) tous deux agencés dans le corps d'atomiseur, dans lequel le cœur d'atomisation comprend une pièce d'atomisation (51) et un corps de guidage de goudron de tabac en forme de bande (52) qui guide du goudron de tabac dans la cavité à goudron de tabac (4) vers une surface d'atomisation de la pièce d'atomisation (51) ; et
le corps de guidage de goudron de tabac en forme de bande (52) se répand à travers la pièce d'atomisation (51) et appuie contre la surface d'atomisation de la pièce d'atomisation (51) et la zone d'une surface de contact du corps de guidage de goudron de tabac en forme de bande (51) et la pièce d'atomisation (51) est inférieure à la zone de la surface d'atomisation de la pièce d'atomisation (51),
dans lequel le corps d'atomiseur comprend une coque (1), une entrée d'air (2), une buse d'aspiration (3) installée en haut de la coque (1) et une électrode de liaison (6) installée au niveau de la partie inférieure de la coque (1) et la cavité à goudron de tabac (4) et le cœur d'atomisation sont agencés dans la coque (1) ;
un passage d'entrée d'air qui communique avec l'entrée d'air (2) est agencé dans la coque (1) et le passage d'entrée d'air conduit à la surface d'atomisation de la pièce d'atomisation (51) ; et un passage de sortie d'air qui communique avec la buse d'aspiration (3) est agencé dans la coque (1) ; et
la pièce d'atomisation (51) est reliée électriquement avec l'électrode de liaison (6), **caractérisé en ce que** le cœur d'atomisation comprend en outre un manchon de fixation (7) agencé pour s'appuyer contre la paroi interne de la coque (1) et situé en dessous de la cavité à goudron de tabac (4) et une première cavité creuse (71) est agencée dans le manchon de fixation (7) ; et
un siège de fixation de pièce d'atomisation (58) est agencé au niveau de la partie inférieure de la première cavité creuse (71) et la pièce d'atomisation (51) est installée sur le siège de fixation de pièce d'atomisation (58) ; et la surface d'atomisation de la pièce d'atomisation (51) appuie contre le corps de guidage de goudron de tabac en forme de bande (52) et une surface de non-atomisation de la pièce d'atomisation (51) est reliée électriquement à l'électrode de liaison (6),
des rainures de fixation (72) sont formées de manière symétrique dans la partie externe du manchon de fixation (7), des trous traversants (73) sont formés latéralement dans les extrémités inférieures des rainures de fixation (72) dans la paroi externe du manchon de fixation (7), le corps de guidage de goudron de tabac en forme de bande (52) pénètre à travers les trous traversants (53), le segment central du corps de guidage de goudron de tabac en forme de bande (52) appuie contre la surface d'atomisation de la pièce d'atomisation (51) et les segments restants des deux extrémités du corps de guidage de goudron de tabac en forme de bande (52) sont serrés dans les rainures de fixation (72) ; et
les deux extrémités du corps de guidage de goudron de tabac en forme de bande (52) s'étirent à partir des rainures de fixation (72) et sont insérées dans la cavité à goudron de tabac (4).

2. Atomiseur selon la revendication 1, dans lequel l'entrée d'air (2) est située au niveau de la partie inférieure de l'électrode de liaison (6), le passage d'entrée d'air est une seconde cavité creuse (61) agencée dans l'électrode de liaison (6) et la seconde cavité creuse (61) communique avec la première cavité creuse (71) ; et
le passage de sortie d'air comprend une conduite d'air (5), le dessus de la conduite d'air (5) communique avec la buse d'aspiration (3), un couvercle d'étanchéité creux (53) est agencé au niveau de l'extrémité inférieure de la conduite d'air (5) et le couvercle d'étanchéité (53) est relié à la partie supérieure de la première cavité creuse (71) et scelle de manière étanche la partie supérieure de la première cavité creuse (71).

3. Atomiseur selon la revendication 1, dans lequel une baïonnette de fixation (55) est formée dans le siège de fixation de pièce d'atomisation (58) et le coin du corps de guidage de goudron de tabac en forme de bande (52) est serré et fixé dans la baïonnette de fixation (55).

4. Atomiseur selon la revendication 1, dans lequel le corps de guidage de goudron de tabac en forme de bande (52) est un fil de fibre de verre présentant un diamètre de 0,3 à 5 mm.

5. Atomiseur selon l'une quelconque des revendications 1 à 4, dans lequel la zone de la surface de contact du corps de guidage de goudron de tabac en forme de bande (52) et de la pièce d'atomisation (51) représente 5 % à 25 % de la zone de la surface d'atomisation de la pièce d'atomisation (51).

6. Atomiseur selon la revendication 1, dans lequel la pièce d'atomisation (51) est une pièce en céramique piézoélectrique solide.

7. Cigarette électronique, comprenant une source de puissance externe, dans laquelle la cigarette électronique comprend en outre l'atomiseur selon l'une quelconque des revendications 1 à 6 et la source de puissance externe est reliée à l'électrode de liaison (6) de l'atomiseur.
